# EUROPEAN PATENT APPLICATION

(11) **EP 4 169 473 A1**
(43) Date of publication of application: **26.04.2023**
(21) Application number: 21204572.8
(22) Date of filing: 25.10.2021
(51) Int. Cl.: A61B 90/00, A61B 17/00, A61B 34/20

(54) **APPARATUS AND METHOD FOR REGISTERING LIVE AND SCAN IMAGES**

(71) Applicant: Erbe Vision GmbH, 78573 Wurmlingen (DE)
(72) Inventor: Shirish, Joshi, 78573 Wurmlingen (DE); Subhamoy, Mandal, 85356 Freising (DE); Faisal, Kalim, 78532 Tuttlingen (DE)
(74) Representative: Rüger Abel Patentanwälte PartGmbB

(57) **Abstract**

The inventive system uses medical imaging and live imaging on a patient's non-rigid tissue structures and increases preciseness and reliability by shaping the patient's body (18) during surgery so that the outer shape of the patient's body during surgery is identical to the outer shape of the body during imaging. The processing unit will precisely overlay a scan image (or an image or a graphical representation derived from several scan images) and a live image acquired during surgery for enhancing surgeons understanding and orientation during surgery. The system comprises a patient support device (11) adaptive to the shape of the patient's body and at least one tracker element (21) connected to the support device (11) and adapted to indicate the position and orientation of the support device (11).

## Description

The invention relates to an arrangement for surgery on a patient's body and a method for image registering.

US patent no. US 9,433,387 B2 discloses a system for obtaining cranial or other scans from a patient. The system comprises a diagnostic scanning table and a mold that conforms to a portion of a patient's body contour. In one embodiment a mask for encompassing the patient's head is provided. Sensors are placed within the mold or mask for obtaining pressure readings, which may indicate movements of the patient such as a swallowing action. The signals of the sensors may be used for avoiding inaccuracies during the scan due to movements of the patient. For doing so, the sensors are coupled to the scanning system.

Furthermore, US 2019/0105423 A1 discloses a support for a patient's limb, which support comprises a network of flexible multi lumen tubing interlaces, which form a lattice structure. By inflating this multi lumen tubing a limb placed within the support may be immobilized.

The article V. Edward, C. Windishburger at al., Quantification of fMRI Artefact Reduction by a Novel Plaster Cast Head Holder (published online September 2000, Wiley-Liz, Inc. discloses a plaster cast head holder for immobilization and repositioning a patient's head during an fMRI scan. The plaster cast head holder will decrease the magnitude of unintentional head movements and reduce movement artefacts. The plaster cast head holder comprises a head mask with malleable fixation material which is stiff in its fully hardened state.

While there is a wide variety of techniques for acquiring images from a patient's body before surgery as there are ultrasonic imaging radioscopy, computer tomography (CT) or magneto resonance imaging (MRI), the surgeon may still have problems with identifying objects and tissue viewed with a camera during surgery. Therefore, there is a strong desire to obviate this problem and help the surgeon orientate during surgery.

This objective is solved by the arrangement according claim 1 and by the method according to claim 15 as well.

The inventive arrangement comprises a patient support device adaptive to the shape of the patient's body. The support device as whole or at least the part(s) adapted to the patient's body may be used during (preferably preoperative) medical imaging and during surgery as well. In a first embodiment the patient support device may be produced as a rigid body shaping element e.g. by three dimensional printing (3D-printing) according to a scan of the shape of a body part of the patient. The shape scan can be performed before any medical scan or live scan is performed and acquires the outer shape of at least a part of the patient's body. Alternatively, the shape of the patient's body part can be directly obtained from a medical scan without the necessity of performing a shape scan beforehand, e.g. utilizing an automatic skin segmentation of the body part in the medical scan. Obtaining the shape of the patient's body part directly from a medical scan can reduce the risk of further changes of the position of the patient's body part in between the shape scan and the medical scan. A mechanical scanner, a laser scanner, a camera system, or any other suitable shape acquiring device can be used for scanning the shape of the body or at least a part thereof. The so acquired data characterizing the patient's body's shape can be used for producing the rigid body shaping element applied to the patient during the medical scan and during surgery as well.

In a second embodiment, the patient support device itself is adapted to capture the contour of at least a region of a patient during the preoperative scan and keep this contour afterwards. The patient support device or at least a contour capturing part of the device may have two states, an initial (soft) state in which it may adapt to the shape of the body, and a final (hardened) state in which it keeps the shape once acquired at or on the patient's body. The patient support device will be used for positioning the patient during the surgical procedure in the same shape as during the medical imaging (during the scan). The at least one tracker element connected to the support device is adapted to indicate the position and orientation of the support device. In other words, the tracker element is adapted to indicate the location (X, Y, Z) of at least one point in space of the support in combination with three orientations (angular orientation around axes X, Y and Z). Alternatively, two or more tracker elements may be provided for indicating the spatial position and orientation of the support device.

Furthermore, the arrangement comprises means for capturing data indicating the position and/or orientation of any of the tracker elements, e.g. cameras, a CT-scanner, an X-ray apparatus or any other arrangement or medical imaging device. The medical imaging device is adapted to acquire at least one at least two dimensional scan image of a patient's region of interest. The medical imaging device is adapted to acquire those images in relation to the position of the at least one tracker element. The images may be acquired in a pre-operative scan or even during surgery. The trackers attached to the support device may be localized therewith. The medical imaging device may comprise a processing unit, which generates the scan image within a coordinate system with the tracker elements located in the same coordinate system.

At the operation site a localization system is provided for capturing data indicating the tracker's position (and/or orientation) during surgery. The data regarding the position of the trackers may be captured by at least one tracking camera. The camera might not be close to the surgical site but kept at a distance. However, line of sight visibility should be ensured.

Alternatively an active tracking method may be used where the support device may emit at least one signal, such as a radio frequency (RF), a light or ultrasonic signal. The signal of the support device can be received and used for capturing/obtaining the support device's position (and/or orientation) during surgery. For example, RF trackers can be embedded in the support device. In that case the data is captured/transmitted from the site the trackers are placed.

Since the patient is placed within the same individually shaped portion of the support device as he or she was during the medical imaging process the patient's body will assume the same shape during surgery as it had during the medical scan. Therefore, the data captured by the localization system precisely indicates the position of the patient support device and hence the positions of tissue structures of the patient. The shape adaptive support device will now reshape the patient's body and make sure that all tissues of the patient are in the same place as they have been during the medical scan. This is in particular important for surgical operations on or in soft tissues with high flexibility and with no specific natural or artificial land marks.

The processing unit may register and blend the scan image and the live image and provide perfect orientation for the surgeon. In particular, the inventive system provides the following advantages:
1. There is no need for preoperative interventions for implanting tracking markers or markings inside the patient's body.
2. There is no need for using screws, pins or the like in bones or target structures. Therefore, additional surgical procedures and complications can be avoided.
3. There is reduced or even no need for using additional medical imaging devices during the surgical operation.
4. The inventive method and system does not rely on rigid anatomies (as there are bones) or organs with low deformation.

The invention avoids changes in the contour of a patient's torso due to redistribution of the weight caused by changing the patient's contour or position. Therefore, the invention allows for acquiring medical images and performing the surgical procedure afterwards. The surgical procedure may be performed in a position of the patient different from the position during medical imaging.

Placing the patient in or on a patient support device adaptive to the shape of the patient's body will transfer the patient body's shape to the support device. The support device will capture and keep the contours of the patient during the preoperative scan. The support device may comprise at least one element comprising casting material, e.g. plaster cast, fiber glass, resin-based casts or anything else, which material is malleable when the patient first is placed thereon or therein. After becoming rigid the shaped support device (the cast) will be reused while repositioning the patient during the surgical procedure. At least one tracker element will be placed on or in the support device before or after curing the malleable curable material. Embedded elements like screws, fiducials, balls, pins or the like may be used as trackers and will act as positional markers to register the patient to the navigational system. The navigational system may involve the processing unit adapted to register the at least one scan image with the at least one live image based on the markers placed in or on the support device.

The patient support device may involve a movable table with a deformable element placed thereon. The deformable element may be removably connected to the table. The deformable element may have two states, an initial (soft) state in which it may adapt to the shape of the patient's body, and a final (hardened) state in which it keeps the shape once acquired at or on the patient's body. So the deformable element can be moved from one table used for scanning the patient to another support for surgery. Since the at least one tracker element is firmly connected to the deformable element and since the deformable element will be transformed from a deformable state into a rigid state after the patient was placed therein and before the medical scan is done, the navigation system will find tissues and organs in the surgical site in the same spatial relationship to the at least one tracker element as they have been during medical imaging.

Preferably, the at least one deformable element is open at one side so that the patient can be removed from, and reinserted into, the deformable and curable element. Moreover, the support device may comprise at least two deformable elements, which when placed around the patient or a portion thereof, will encompass and hence embrace the patient or a portion thereof. This is in particular useful if the medical imaging and the surgery will be performed at different points of time e.g. on different days. In particular, if the surgery is to be done on body portions with little or no rigid structures, the adaptive patient support device after assuming the shape of the patient and hardened in this shape will reshape the patient's body and safeguard that the flexible tissues and organs assume the same positions as in the imaging step before, if the patient reenters the device.

The inventive arrangement may involve an instrument for treating the patient's body in the region of interest. The instrument may be any type of RF surgical, cryosurgical, plasma surgical or any other instrument. In particular, the instrument is adapted to be placed within the field of view of the live imaging device. The live imaging device may be part of the instrument, a separate camera inserted into a trocar, or a separate camera placed at, on, or in the patient's body.

The tracker system is placed at the surgical site of the arrangement and comprises at least two cameras or other positional detectors for trigonometrically determining the place (and if necessary the orientation) of the at least one tracker element in space. Moreover, the imaging system may comprise at least one tracker element or any other sensor connected to a detection system adapted to detect location and orientation of the camera. The detection system may be connected to the processing unit, which accordingly may register the live image and the scan image.

Furthermore, the processing unit may comprise a structure detecting unit for generating graphical representations of tissue structures, which graphic representations may be obtained from the medical images. The graphic representations may be lines, symbols, colored areas or anything else, adapted to indicate regions or tissues the surgeon shall distinguish from other regions or tissues. These graphic structures may be blended into the live image, which helps the surgeon to find structures to be treated within the region of interest.

Further details and features may be found in the drawing, the description and claims as well.
Figure 1 is a schematic representation of a patient support device with a patient placed thereon and a medical imaging device,
Figure 2 is a cross-sectional view of the patient support device and the patient placed thereon,
Figure 3 is a schematic representation of scan image provided by the medical imaging device,
Figure 4 is the patient according to Figure 1 placed on a patient support device at the operation site of the inventive arrangement, including a localization system for localizing the patient during surgery,
Figure 5 illustrates a scan image, a live image and blended image provided to the surgeon,
Figure 6 is a schematic representation of the camera for acquiring live images, and
Figure 7 illustrates the scan images, a volume model of a patient's tissue structure obtained from the scan images, and the live image registered into the spatial representation of the tissue structure.

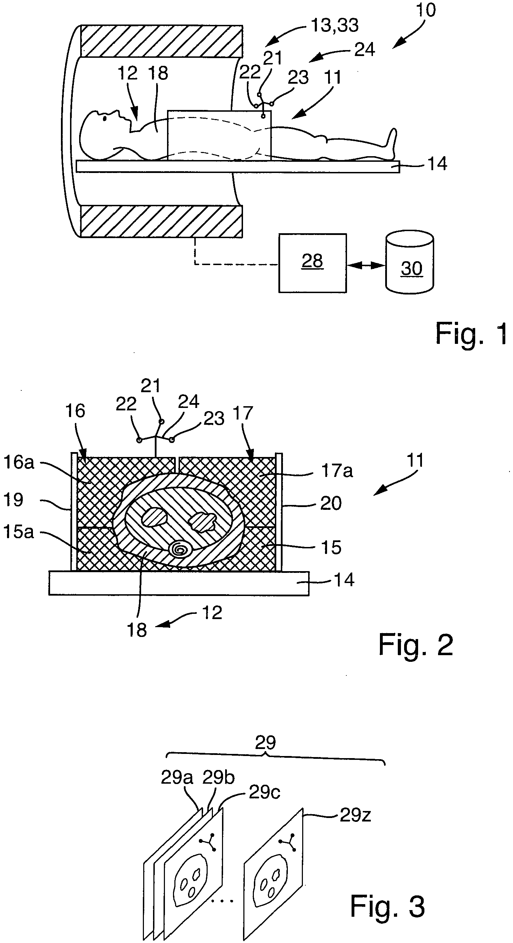

Figure 1 illustrates an arrangement for medical imaging 10, comprising a support device 11 for positioning a patient 12 in a desired position relative to a medical imaging system 13. The support device 11 may consist of a table 14 and at least one deformable element 15 placed thereon. Further deformable elements 16 and 17 may be placed around the patient's 12 body 18 as can be taken from Figure 2 illustrating a cross-sectional view of the table 14, the deformable elements 15 to 17, and the body 18.

The deformable elements 15 to 17 may be cushions filled with malleable durable material as there is plaster cast, fiber glass, reinforced plaster cast, resin-based casts or the like. Alternatively the malleable material may be directly placed on the skin of the patient or at a cloth lying on the skin. While the deformable element 15 may be placed between the body 18 of the patient 12 and the table 14 elements 16 and 17 may be placed on the patient 12 and e.g. held between walls 19, 20. At least one of the deformable elements 15 to 17 and/or at least one of the walls 19 is firmly connected to tracker elements 21, 22, 23, which in the present case are balls fixed at the ends of a tree 24 in known distances one from another. Balls 21 to 23 may be light-reflecting or light-absorbing balls visible by the imaging system 13 or a specific camera assigned to the imaging system 13. In the present case three tracking elements are provided for unambiguously indicating the location and orientation of the patient 12. However, while three balls 21, 22, 23 placed on the ends of a tree give a fairly good indication of the location and orientation of the patient 12, it is also possible to place at least three different balls independent one from another at different places of the deformable element 15, 16 or 17. If the tracker elements 21, 22, 23 will be optically detected they will be placed at a visible side of the deformable elements 15 to 17.

Furthermore, it is possible to use only one item as a tracker element, e.g. one cube firmly connected to the support 14 or at least one of the deformable elements 15, 16 and 17.

The imaging system 13 can be any type of medical imaging systems for acquiring a pre-operative medical scan as there is a MRI-system or a CT system as illustrated in Figure 1. The CT system may comprise an X-ray source and an X-ray detector adapted to receive X-ray from source and deliver data to a processing unit 28 producing scan images 29 (29a to 29z) as illustrated in Figure 3. The processing unit 28 may be any type of computer adapted to process signals supplied by the X-ray detector 27. The processing unit 28 is connected to a storage 30 for storing the scan images 29 therein. Alternatively or additionally an intraoperative scan apparatus may be provided, e.g. a C-Arm system, an ultrasonic imaging apparatus or any other system suited for providing medical scan images during operation.

The medical imaging system 13 may additionally be used as a means for capturing data 33 indicating the positions of the tracking elements 21, 22, 23 during operation of the imaging system i.e. during the scanning of the patient's body 18. Alternatively a separate localization system may be provided for detecting and locating the tracking elements 21, 22, 23 and bringing the images 29 into spatial relation to the tracking elements 21, 22, 23.

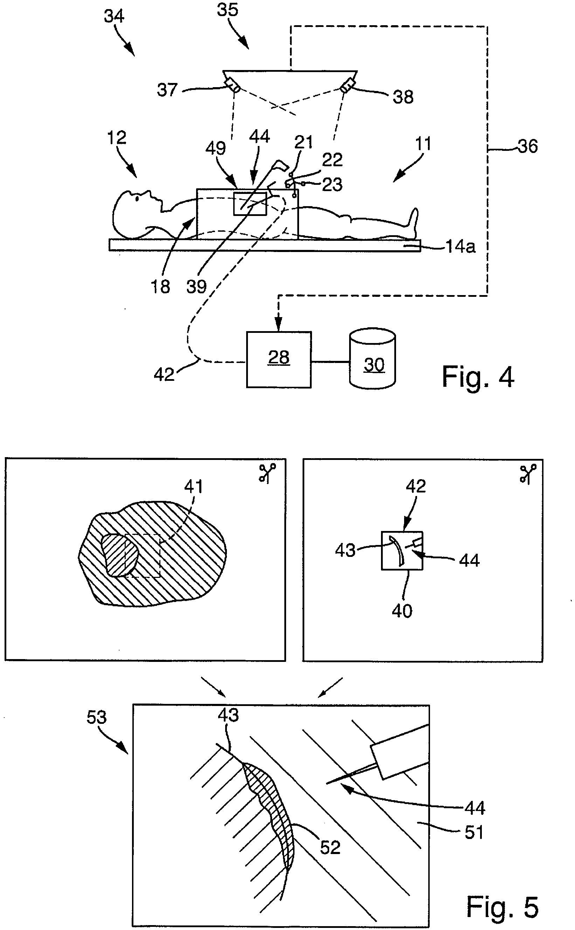

Part of the inventive arrangement 10 is an operation site 34 illustrated in Figure 4. The patient 12 again is placed on a table 14a, which may be identical with the table 14 of the imaging site illustrated in Figure 1. However, typically table 14a will be a different table as typically used in a regular operating room. No matter whether the tables 14, 14a are identical or not, in any case the deformable elements 15 to 17 will be used for presenting the patient's body 18 in the same shape as it has had during medical imaging when illustrated in Figure 2. Moreover the tracking elements 21, 22, 23 will be in the same position relative to the patient's body 18 during imaging and during surgery as well.

At the operation site 34 a localization system 35 is provided for capturing data 36 fed to a processing unit 28a connected to storage 30. The processing unit 28a may be identical with processing unit 28 of Figure 1 or alternatively it may be a different processing unit. Processing unit 28a may be any type of computer or processor adapted to receive data from the localization system 35 and determine the position and orientation of the tracker elements 21, 22, 23 and hence the position and orientation of the patient's 12 body 18. The localization system 35 may comprise at least two cameras 37, 38 oriented such that the tracking elements 21, 22, 23 are within the field of view of the cameras 37, 38. The processing unit 28 or 28a is adapted to locate the tracker elements 21, 22, 23 by triangulation once before the surgery starts if the table 14a is kept at rest. If table 14a is moved the localization system 35 may repeat determining the localization and orientation of the patient's 12 body 18. Alternatively, the localization may be done permanently by the localization system 35.

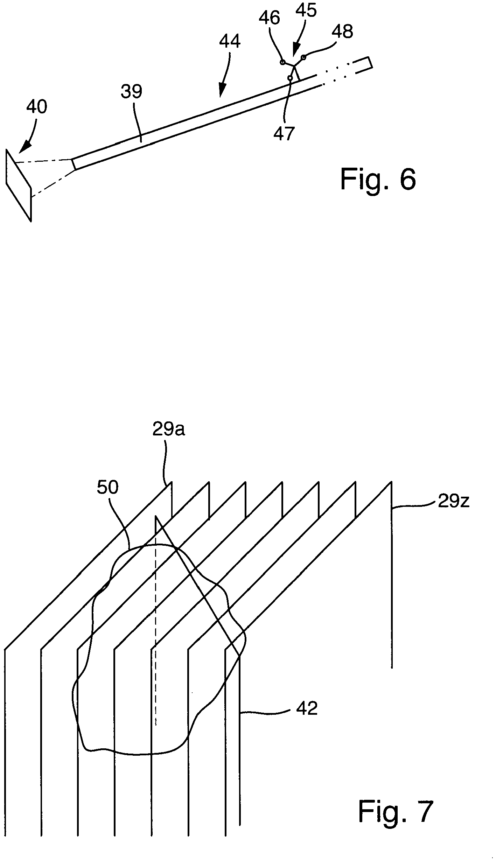

Part of the arrangement is another camera 39 for acquiring live images as separately illustrated in Figures 4 and 6. The field of view 40 of the camera 39 is a region of interest 41 of the patient's body 18 at which a surgery is to be performed. Figure 5 illustrates region of interest 41 covered by the field of view 40. The camera 39 may be a laparoscopic camera, and endoscopic camera or any other type of camera suitable and adapted to produce a live image 42 of the region of interest 40.

The live image 42 may be fed to the processing unit 28 or 28a as illustrated in Figure 4. The processing unit 28a may process any live image 42 which is shown in Figure 5, right upper illustration. The Live image 42 may contain a real tissue structure 43 and the tip of an instrument 44.

Any type of localization system may be used for detecting location and orientation of the instrument 44 and/or the camera 39. The localization system 35 may involve a tracker structure 45 connected to the camera 39 and visible by the cameras 37, 38. The tracker structure may comprise at least one tracker element, e.g. three tracker elements 46, 47, 48 as illustrated in Figure 6, similar to the tracker elements 21, 22, 23. Other types of tracking systems may be used.

The so far described arrangement 10 operates as follows:

Before surgery the patient 12 will be placed on the support 11 device at the table 14 with the deformable element 15 shaped by the body 18 as illustrated in Figure 2. If desired or necessary, one or two further deformable elements 16, 17 will be placed around the patient's body 18 so that the deformable elements 15, 16, 17 assume the negative shape of the patient's body 18 and fit closely around the body 18. The deformable elements 15, 16, 17 are filled with or formed by malleable material which will solidify over time, e.g. within some minutes or some tens of minutes. After curing i.e., solidifying the imaging system 13 may acquire scan images 29a through 29z, which images are stored by processing unit 28 in the storage 30. Afterwards the patient 12 may leave the support device 11 and prepare for surgery, which may follow within short and sometimes after hours or days.

For surgery the patient 12 reenters the support device 11 as illustrated in Figure 4 by placing his or her body 18 at the table 14a with the once deformable and now rigid element 15 to 17, placed around the body 18 as illustrated in Figure 2. The surgeon may have cut-out a window 49 in one or more of the elements 15, 16, 17 so that he or she has access to the body 18 through window 49. The window 49 may also be provided in the support device, in particular in the deformable element(s) 15, 16, 17 for planned operation before the patient is placed in the deformable element. Alternatively the window 49 may be cut into the deformable element(s) 15, 16, 17 between the medical pre-operative scan and the surgery. This process may be part of planning the operation.

At the beginning or before the beginning of the surgery the localization system 35 will be activated, which captures the positions of the tracking elements 21, 22, 23. So the processing unit 28a may register the position of the patient's body 18 to the scan images 29a to 29z as illustrated in Figure 7. Moreover, the processing unit 28a or the processing unit 28 may produce a volume model 50 of at least a portion of the patient's body, e.g. of the region of interest 41.

The localization system 35 or any other tracking system for determining the position and orientation of the camera 39 continuously produces data from which the processing unit 28a determines the place and orientation of the field of view 40 of the camera 39 and hence the place of the live image 42 and the viewing direction to the live image. As illustrated in Figure 7, the live image 42 may intersect the volume model 50 in a different way as do the scan images 29a to 29z. However, the processing unit 28a may produce a synthetic image of the volume model 50 as illustrated in Figure 5 upper left illustration at least of the region of interest 41. For doing so the processing unit may intersect the volume model 50 in the same plane as the live image.

The processing unit 28a will then merge or blend the live image 42 (Figure 5 upper right illustration) with the volume model illustration derived by intersecting the volume model 50 at the same place and with the same orientation as has the live image 42. Figure 5 illustrates the blended image 51 with the tissue structures 43 seen by camera 39 and a specific tissue structure 52 found by the imaging and to be treated by instrument 44.

Furthermore the processing unit 28 or 28a may alternatively or additionally produce graphic representations 52 of tissue structures and blend those graphic representations into the live image. Any of the scan images 29, the image obtained by intersecting the volume model 50, and graphic representation 52 obtained from at least one of the scan images or from the volume model 50 are considered being "a scan image" for blending with the "live image" according to claim 1. The arrangement 10 further comprises an image display 53 for reproducing the blended image. The display 53 may be a screen, a virtual reality headset or any other means for showing the blended image.

The inventive system uses medical imaging and live imaging on a patient's non-rigid tissue structures and increases preciseness and reliability by shaping the patient's body 18 during surgery so that the outer shape of the patient's body during surgery is identical to the outer shape of the body during imaging. The processing unit 28 or 28a will precisely overlay a scan image (or an image or a graphical representation derived from several scan images) and a live image acquired during surgery for enhancing surgeons understanding and orientation during surgery.

### Reference Numerals:

- 10: Arrangement for medical imaging
- 11: Support device
- 12: Patient
- 13: Imaging system
- 14, 14a: Table
- 15 - 17: Deformable element
- 15a - 17a: Malleable / curable material
- 18: Patient's body
- 19, 20: Walls
- 21 - 23: Tracker elements
- 24: Tree
- 28, 28a: Processing unit
- 29: Scan images
- 30: Storage
- 33: Means for capturing data
- 34: Operation site
- 35: Localization system
- 36: Data
- 37, 38: Cameras
- 39: Camera
- 40: Field of view of camera 39
- 41: Region of interest
- 42: Live image
- 43: Tissue structure
- 44: Instrument
- 45: Tracker structure
- 46 - 48: Tracker elements
- 49: Window
- 50: Volume model
- 51: Blended image
- 52: Tissue structure
- 53: Display

## Claims

1. An arrangement for imaging (10) and surgery (34) on a patient's body (18) comprising:
A patient support device (11) adaptive to the shape of the patient's body,
At least one tracker element (21) connected to the support device (11) and adapted to indicate the position and orientation of the support device (11),
Means for capturing data (33) indicating the position and/or orientation of the at least one tracker element (21),
A medical imaging system (13) adapted to acquire at least one at least 2-dimensional scan image (29) of a patient's region of interest in relation to the position of the at least one tracker element (21),
A localization system (35) for capturing data (36) indicating the at least one tracker's position during surgery,
A live imaging device (39) for acquiring live images (42) of the operation site,
A processing unit (28, 28a) adapted to register and blend the scan and live images (29, 42, 52) according to the data captured during the medical imaging and live imaging.

2. The arrangement of claim 1, wherein the patient support device (11) involves a movable table (14, 14a).

3. The arrangement of any of the preceding claims, wherein the patient support device (11) is adaptive to the shape of the patient's body by 3D-printing at least one element (15) according to a scan of at least a part of the outer shape of the patient's body.

4. The arrangement of any of claims 1 or 2, wherein the patient support device (11) comprises a deformable element (15) involves or consists of a malleable material (15a), and wherein the malleable material is a curable material (15a).

5. The arrangement of claim 3, wherein at least one tracker element (21) is directly connected to the at least one deformable element (15).

6. The arrangement of claim 3, wherein the at least one deformable element (15) is open at one side, so that the patient can be removed from and reinserted into the deformable element (15).

7. The arrangement of any of the preceding claims, wherein the support device comprises at least two deformable elements (15, 16, 17) embracing at least a portion of the patient.

8. The arrangement according to claim 6 or 7, wherein the at least one deformable element (15, 16, 17) comprises a window (49) for giving access to the patient.

9. The arrangement of any of the preceding claims, comprising an instrument (44) for treating the patient's body (18) in the region of interest (41).

10. The arrangement of any of the preceding claims, wherein the tracker element (21) is adapted to indicate the place and the orientation of the support device in space.

11. The arrangement of any of the preceding claims, wherein the tracker element (21) comprises spaced apart reflector elements localizable by the localization system.

12. The arrangement of any of the preceding claims, wherein the localization system comprises at least two cameras (37, 38) for trigonometrically determining the place and the orientation of the at least one tracker element (21) in space.

13. The arrangement of any of the preceding claims, wherein the live imaging device comprises at least one camera (39).

14. The arrangement of claim 13, wherein a detection system (35) provided for detecting location and orientation of the camera (39) of the live imaging device.

15. Method for image registering comprising:
Adapting a patient support device (11) to the shape of the patient's body (18),
Connecting at least one tracker element (21) to the support device (11) and capturing data indicating the position and/or orientation of the at least one tracker element (21),
Acquiring at least one at least 2-dimensional scan image (29) of a patient's region of interest (41) in relation to the position of the tracker (21) by using a medical imaging system (13),
Capturing data (36) indicating the tracker's position during surgery by means of a localization system (35),
Acquiring live images (42) of the operation site by a live imaging device (39),
Registering and blending the scan and live images according to the data (36) captured during the medical imaging and live imaging.
